# FASCICULE DE BREVET EUROPEEN

(11) **EP 2 300 421 B1**
(45) Date de publication et mention de la délivrance du brevet: **02.12.2015**
(21) Numéro de dépôt: 09753936.5
(22) Date de dépôt: 28.05.2009
(51) Int. Cl.: C07C 303/28, C07C 309/65

(54) **PROCEDE DE SULFONYLATION D'UN COMPOSE ORGANIQUE HYDROXYLE**
VERFAHREN ZUR SULFONYLIERUNG EINER HYDROXYLIERTEN ORGANISCHEN VERBINDUNG
METHOD FOR THE SULPHONYLATION OF A HYDROXYLATED ORGANIC COMPOUND

(30) Priorité: 29.05.2008 FR 0802943
(43) Date de publication de la demande: 30.03.2011
(73) Titulaire: Rhodia Opérations, 93306 Aubervilliers (FR)
(72) Inventeur: METZ, François, F-69540 Irigny (FR)
(74) Mandataire: Dutruc-Rosset, Marie-Claude
(86) Numéro de dépôt international: PCT/EP2009/056554
(87) Numéro de publication internationale: WO 2009/144281

(56) Documents cités:
- EP-A- 1 288 198
- FR-A- 2 031 455
- FR-A- 2 470 119
- US-A- 3 346 612

## Description

La présente invention a pour objet un procédé de sulfonylation d'un composé organique hydroxylé.

L'invention concerne plus particulèrement un procédé de trifluorométhanesulfonylation d'un composé organique hydroxylé.

L'invention vise notamment les composés hydroxylés de type aliphatique et plus particulièrement ceux qui comprennent une chaîne aliphatique perfluorée.

L'invention s'applique préférentiellement aux alcools aliphatiques perfluorés et, notamment, au 2,2,2-trifluoroéthanol.

Il est connu selon FR-A 1 470 669 de préparer le triflate de trifluoroéthyle (CF₃SO₃CH₂CF₃) appelé de manière simplifiée « TfOTFE » par réaction du 2,2,2-trifluoroéthanol avec le chlorure de triflyle (CF₃SO₂Cl), en présence d'une base telle que la triéthylamine et dans un solvant organique qui est le dichlorométhane.

Il a été proposé dans US 3 346 612 un procédé de préparation d'aryles perfluoroalcanes-sulfonates à partir d'un phénol et d'un agent de sulfonylation tel qu'un halogénure d'un acide perfluoroalkylsuifonique en présence d'une base organique telle que la pyridine, les pipéridines, les phénoxydes de métaux alcalins, et plus particulièrement les triéthylamines.

L'inconvénient dont souffre ce procédé est qu'il conduit à des rejets très polluants dus à la présence de fortes quantités de sel d'ammonium.

Il a été proposé selon EP-A 1 322 601, un procédé de sulfonylation d'un composé organique hydroxylé qui consiste à faire réagir ledit composé, avec un agent de sulfonylation, en présence d'une quantité efficace d'un acide de Lewis.

L'acide de Lewis est un composé comprenant un cation métallique ou métalloïdique considéré comme « intermédiaire » dans la classification « dureté (hard) et mollesse (soft) » définie par R. PEARSON. Le chlorure d'antimoine est un exemple d'acide de Lewis convenant au procédé décrit dans EP-A 1 322 601.

Bien que cette catalyse soit très intéressante, la Demanderesse était à la recherche d'un procédé faisant appel à des réactifs plus courants.

L'objectif de la présente invention est de fournir un procédé permettant d'éviter les inconvénients précités.

Il a maintenant été trouvé et c'est ce qui fait l'objet de la présente invention, un procédé de trifluorométhanesulfonylation d'un composé organique hydroxylé caractérisé par le fait qu'il comprend la réaction dudit composé, avec un agent de trifluorométhanesulfonylation, en milieu organique, en présence d'une base minérale hétérogène et d'un solvant organique apolaire.

Le composé organique hydroxylé qui intervient dans le procédé de l'invention répond plus particulièrement à la formule (I) :

R₁-O-H (I)

dans ladite formule (I) :
- R₁ représente un groupe hydrocarboné, substitué ou non, comportant de 1 à 40 atomes de carbone qui peut être un groupe aliphatique acyclique saturé ou insaturé, linéaire ou ramifié ; un groupe carbocyclique ou hétérocyclique saturé, insaturé ou aromatique, monocyclique ou polycyclique ; un enchaînement des groupes précités.

Plus précisément, R₁ représente un groupe hydrocarboné ayant de 1 à 20 atomes de carbone qui peut être un groupe aliphatique acyclique saturé ou insaturé, linéaire ou ramifié ; un groupe carbocyclique ou hétérocyclique saturé, insaturé ou aromatique, monocyclique ou polycyclique ; un groupe aliphatique saturé ou insaturé, linéaire ou ramifié, porteur d'un substituant cyclique.

R₁ représente préférentiellement un groupe aliphatique acyclique saturé linéaire ou ramifié ayant de préférence de 1 à 12 atomes de carbone, et encore plus préférentiellement de 1 à 4 atomes de carbone.

L'invention n'exclut pas la présence d'une insaturation sur la chaîne hydrocarbonée telle qu'une ou plusieurs doubles liaisons qui peuvent être conjuguées ou non, ou une triple liaison.

La chaîne hydrocarbonée peut être éventuellement interrompue par un hétéroatome (par exemple, oxygène ou soufre) ou par un groupe fonctionnel dans la mesure où celui-ci ne réagit pas et l'on peut citer en particulier un groupe tel que notamment -CO-.

La chaîne hydrocarbonée peut être éventuellement porteuse d'un ou plusieurs substituants (par exemple, halogène, ester, aldéhyde) dans la mesure où ils n'interfèrent pas avec la réaction de sulfonylation.

Ainsi, la chaîne hydrocarbonée porte préférentiellement un ou plusieurs atomes de fluor.

Le groupe aliphatique acyclique, saturé ou insaturé, linéaire ou ramifié peut être éventuellement porteur d'un substituant cyclique. Par cycle, on entend un cycle carbocyclique ou hétérocyclique, saturé, insaturé ou aromatique.

Le groupe aliphatique acyclique peut être relié au cycle par un lien valentiel, un hétéroatome ou un groupe fonctionnel tels que oxy, carbonyle, carboxy ou sulfonyle.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs d'un substituant quelconque dans la mesure où ils ne gênent pas les réactions intervenant dans le procédé de l'invention. On peut mentionner en particulier, les groupes alkyle, alkoxy ayant de 1 à 4 atomes de carbone.

Parmi les groupes aliphatiques porteurs d'un substituant cyclique, on vise plus particulièrement les groupes aralkyle ayant de 7 à 12 atomes de carbone, notamment benzyle ou phényléthyle.

Dans la formule (I), R₁ peut également représenter un groupe carbocyclique, saturé ou non ayant de préférence 5 ou 6 atomes de carbone dans le cycle ; un groupe hétérocyclique, saturé ou non, comportant notamment 5 ou 6 atomes dans le cycle dont 1 ou 2 hétéroatomes tels que les atomes d'azote, de soufre et d'oxygène ; un groupe carbocyclique ou hétérocyclique aromatique, monocyclique, de préférence, phényle ou pyridyle ou polycyclique condensé ou non, de préférence, naphtyle.

Dès lors que R₁ comprend un cycle, celui-ci peut être également substitué. Le nombre de substituants est généralement au plus de 4 par cycle mais le plus souvent égal à 1 ou 2. La nature du substituant est indifférente dans la mesure où il ne réagit pas dans les conditions de l'invention, en particulier avec une base forte. Comme exemples de substituant, on peut citer notamment les groupes alkyle, alkoxy ayant de 1 à 4 atomes de carbone ou les atomes d'halogène.

L'invention n'exclut pas le cas où le composé organique hydroxylé est difonctionnel c'est-à-dire qu'il porte un autre groupe OH porté par un chaîne aliphatique ou en tant que substituant d'un cycle aromatique.

Comme exemples plus spécifiques, on peut mentionner l'enchaînement suivant de cycles aromatiques éventuellement porteurs de substituants :

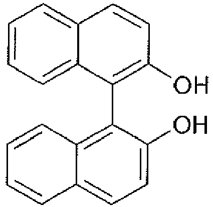

Parmi toutes les significations données précédemment pour R₁, R₁ est de préférence un groupe alkyle linéaire ou ramifié ayant de 1 à 12 atomes de carbone, et de préférence de 1 à 4 atomes de carbone.

Le procédé de l'invention s'applique tout particulièrement aux alcools aliphatiques et plus particulièrement aux alcools fluorés et perfluorés répondant à la formule (la) :

R₁-O-H (la)

dans ladite formule (la), R₁ représente une chaîne alkyle fluorée ou perfluorée comprenant de 1 à 10 atomes de carbone et de 1 à 21 atomes de fluor, de préférence, de 3 à 21 atomes de fluor.

L'invention concerne plus particulièrement les alcools aliphatiques perfluorés répondant à la formule (Ia) dans laquelle R₁ représente une chaîne alkyle perfluorée comprenant de 1 à 10 atomes de carbone et de 3 à 21 atomes de fluor.

Le procédé de l'invention s'applique particulièrement bien aux composés de formule (I) ou (la) tels que l'éthanol, le 2,2,2-trifluoroéthanol, le 2,2-difluoroéthanol, le 1,1-difluoroéthanol, le pentafluoroéthanol, l'hexafluoroisopropanol, le pentafluorophénol, le p-nitrophénol, le p-trifluorométhylphénol.

Pour ce qui est de l'agent de sulfonylation, il s'agit d'un halogénure d'un acide perfluoroalkylsulfonique.

Il répond à la formule suivante (II) : 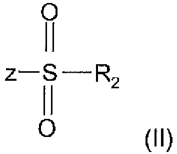 dans ladite formule (II) :
- R₂ représente un groupe hydrocarboné perfluoroalkyle CF₃,
- Z représente un atome de fluor, de chlore ou de brome.

Dans la formule (II), on choisit de préférence le cas où Z est un atome de chlore ou de brome. Z est plus préférentiellement un atome de brome.

L'agent de sulfonylation selon l'invention qui est dénommé « agent de trifluorométhanesulfonylation », répond à la formule (II) dans laquelle R₂ représente un groupe CF₃ et Z représente un atome de fluor, de chlore ou de brome.

Comme exemples préférés d'agents de sulfonylation, on fait donc appel préférentiellement au fluorure de trifluorométhanesulfonyle, au chlorure de trifluorométhanesulfonyle ou au bromure de trifluorométhanesulfonyle.

Le bromure de trifluorométhanesulfonyle est préféré.

Selon le procédé de l'invention, la réaction entre le composé organique hydroxylé et l'agent de sulfonylation est conduite en phase liquide, en présence d'un solvant organique apolaire et d'une base minérale hétérogène.

Le rapport entre le nombre de moles d'agent de sulfonylation et le nombre de moles de composé organique hydroxylé peut varier entre 0,5 et 2, et se situe de préférence entre 0,8 et 1,2.

Intervient dans le procédé de l'invention, une base dont la fonction est de piéger l'acide halohydrique formé par la réaction.

On peut faire appel à un hydroxyde d'ammonium ou d'un métal monovalent et/ou d'un métal bivalent, de préférence un métal alcalin et/ou alcalino-terreux.

Comme exemples plus spécifiques de bases à utiliser, on peut citer un hydroxyde de métal alcalin tel que le sodium, le potassium ou le césium et un hydroxyde de métal alcalino-terreux tel que le magnésium, le calcium, le baryum ; un hydroxyde d'un métal du groupe IIB tel que le zinc.

Dans le présent texte, on se réfère ci-après à la Classification périodique des éléments publiée dans le Bulletin de la Société Chimique de France, n°1 (1966).

Parmi les bases, on choisit préférentiellement l'hydroxyde de sodium ou de potassium.

Une autre classe de bases qui peuvent être utilisées dans le procédé de l'invention est constituée par les carbonates et les hydrogénocarbonates des métaux alcalins ou alcalino-terreux.

On choisit plus préférentiellement le carbonate de sodium ou de potassium.

Selon une caractéristique du procédé de l'invention, on met en oeuvre la base sous une forme solide, généralement sous forme d'une poudre et plus particulièrement sous une forme broyée dans le cas des hydroxydes de métaux alcalins ou alcalino-terreux en particulier dans le cas des hydroxydes de sodium, potassium et lithium.

L'opération de broyage de la base peut être effectuée dans tout type de broyeur résistant à la corrosion de la base (par exemple en inox).

La quantité de base mise en oeuvre est telle que le rapport entre le nombre de moles de base et le nombre de moles d'agent de sulfonylation varie de préférence entre 1 et 2, et plus préférentiellement entre 1,4 et 1,6.

La réaction est conduite en présence d'un solvant organique apolaire.

On choisit un solvant qui soit inerte dans les conditions réactionnelles.

Les solvants appropriés sont les hydrocarbures aliphatiques, cycloaliphatiques ou aromatiques halogénés ou non.

A titre d'exemples non limitatifs de tels solvants, on peut citer les hydrocarbures aliphatiques et cycloaliphatiques plus particulièrement les paraffines tels que notamment, l'hexane, l'heptane, l'octane, l'isooctane, le nonane, le décane, l'undécane, le tétradécane, l'éther de pétrole et le cyclohexane ; les hydrocarbures aromatiques comme notamment le benzène, le toluène, les xylènes, l'éthylbenzène, les diéthylbenzènes, les triméthylbenzènes, le cumène, le pseudocumène, les coupes pétrolières constituées de mélange d'alkylbenzènes notamment les coupes de type Solvesso^{®}.

On peut également faire appel aux hydrocarbures halogénés aliphatiques ou aromatiques, et l'on peut mentionner : les hydrocarbures perchlorés tels que notamment le trichlorométhane, le tétrachloroéthylène ; les hydrocarbures partiellement chlorés tels que le dichlorométhane, le dichloroéthane, le tétrachloroéthane, le trichloroéthylène, le 1-chlorobutane, le 1,2-dichlorobutane ; le rnonochlorobenzène, les dichlorobenzènes ou leurs mélanges ; le trifluorométhylbenzène, le trifluorométhoxybenzène.

Conformément au procédé de l'invention, il a été trouvé d'une manière surprenante, que le rendement de la réaction de sulfonylation, conduite en présence d'une base minérale hétérogène était particulièrement élevé dès lors que l'on faisait appel à un solvant organique apolaire.

Ainsi, les solvants préférés pour la mise en oeuvre du procédé de l'invention sont les xylènes, le chlorobenzène et les dichlorobenzènes ou leurs mélanges.

On peut également utiliser un mélange de solvants organiques polaires.

La quantité de solvant organique apolaire mis en oeuvre est choisie d'une manière préférentielle telle que la concentration pondérale de l'agent de sulfonylation dans le solvant soit comprise entre 5 et 50 %, de préférence entre 20 et 40 %.

Selon une variante préférée du procédé de l'invention, on choisit de travailler dans des conditions anhydres. Toutefois, il est possible de tolérer dans le milieu réactionnel, une faible quantité d'eau, de préférence inférieure à 1 % en poids et plus préférentiellement inférieure à 0,5 % en poids.

La réaction est en général mise en oeuvre à une température comprise entre 0°C et la température de reflux du solvant organique, de préférence à une température comprise entre 15°C et 30°C.

La réaction de sulfonylation est généralement mise en oeuvre sous pression atmosphérique mais de préférence, sous atmosphère contrôlée de gaz inertes. On peut établir une atmosphère de gaz rares, de préférence l'argon mais il est plus économique de faire appel à l'azote. Une pression légèrement supérieure ou inférieure à la pression atmosphérique peut convenir.

Le procédé de l'invention est simple à mettre en oeuvre.

Les réactifs peuvent être introduits selon de nombreuses variantes mais certaines sont préférées.

Un premier mode de réalisation consiste à préparer un pied constitué par le composé organique hydroxylé et la base minérale hétérogène puis d'introduire progressivement, de préférence par coulée, l'agent de sulfonylation introduit dans le solvant organique apolaire.

Un autre mode d'exécution qui présente l'avantage de faciliter l'agitation du milieu réactionnel, est de former un pied comprenant la base minérale hétérogène et le solvant organique apolaire puis d'introduire progressivement, de préférence par co-coulée, l'agent de sulfonylation et le composé organique hydroxylé.

Après maintien sous agitation du milieu réactionnel, à la température choisie, on obtient en fin de réaction un ester sulfonique répondant à la formule suivante :

R₁OSO₂ R₂ (III)

dans ladite formule, R₁ et R₂ ont la signification donnée précédemment.

On récupère le produit obtenu de manière classique. Généralement, on ajoute de l'eau ce qui permet de récupérer en phase aqueuse les sels formés.

On sépare les phases aqueuse et organique.

On recueille la phase organique que l'on lave éventuellement.

On récupère l'ester sulfonique à partir de la phase organique selon les techniques classiquement utilisées.

On peut par exemple effectuer une distillation ou une extraction à l'aide d'un solvant organique choisi de telle sorte qu'il ne forme pas d'azéotrope avec l'eau.

Le procédé de l'invention permet donc d'obtenir l'ester d'une manière simple avec un bon rendement et une bonne sélectivité.

Un autre avantage du procédé de l'invention est qu'il permet de mettre en oeuvre un agent de sulfonylation de type bromure qui présente la caractéristique d'être beaucoup moins volatil qu'un agent de sulfonylation de type chlorure ce qui est particulièrement intéressant d'un point de vue industriel.

Les exemples qui suivent, illustrent l'invention.

Les exemples 1 à 5 correspondent à la sulfonylation du trifluoroéthanol en présence d'une base hétérogène (carbonate de potassium solide) à l'exception de l'exemple 4 où il s'agit d'une solution aqueuse de carbonate de potassium.

L'exemple 6 est relatif à la sulfonylation de l'éthanol en présence d'une base hétérogène.

Les exemples 7 à 10 sont des essais comparatifs dans lesquels la base est une base organique azotée.

L'exemple 11 concerne la sulfonylation de l'éthanol à l'aide de chlorure de trifluorométhanesulfonyle, en présence d'une base hétérogène.

Dans les exemples, les abréviations suivantes signifient :
- TFE : TriFluoroEthanol,
- TFSBr : Bromure de TriFluorométhaneSulfonyle (bromure de triflyle),
- TFSCI : Chlorure de TriFluorométhaneSulfonyle (chlorure de triflyle),
- ACN : ACétoNitrile,
- MCB : MonoChloroBenzène,
- ODCB : OrthoDichloroBenzène.

Le taux de transformation (TT) du bromure ou chlorure de triflyle (CF₃SO₂Br ou CF₃SO₂Cl) correspond au rapport entre le nombre de moles de bromure ou chlorure de triflyle transformées et le nombre de moles de bromure ou chlorure de triflyle introduites.

Le rendement en TfOTFE (RR_{TfOTFE}) correspond au rapport entre le nombre de moles de TfOTFE formées et le nombre de moles de bromure ou chlorure de triflyle introduites.

Le rendement en TfOEt (RR_{TfOEt}) correspond au rapport entre le nombre de moles de TfOEt (triflate d'éthyle) formées et le nombre de moles de bromure ou chlorure de triflyle introduites.

La sélectivité en TfOTFE (RT_{TfOTFE}) correspond au rapport entre le nombre de moles de TfOTFE formées et le nombre de moles de bromure ou chlorure de triflyle transformées.

### Exemples 1 à 5

Dans un réacteur en verre de 150 ml, on introduit sous azote :
- K₂CO₃ : 7 g (50,14 mmol)
- TFE : 3,2 g (32 mmol)
- solvant : 7 g

Le milieu hétérogène résultant est porté sous agitation à la température T mentionné dans le tableau suivant (I), puis le TFSBr (7 g, 32 mmol), dilué par le TFE (3,2 g, 32 mmol) ou par un solvant (7 g), est ajouté pendant un temps t également mentionné dans le tableau (I).

Après ajout, le milieu réactionnel est maintenu à la température T' pendant un temps t'mentionné dans le tableau (I), puis l'on dose les réactifs et les produits obtenus par RMN ¹⁹F.

Les résultats obtenus sont consignés dans le tableau (I).

**Tableau (I)**

| | REACTION | | | | MAINTIEN | | | | |
|---|---|---|---|---|---|---|---|---|---|
| Ex. | Réactifs en pied | T en pied (°C) | Réactifs coulés | Temps de coulée (h) | T' (°C) | t' (h) | TT (%) | RR (%) | RT (%) |
| 1 (comp.) | K₂CO₃/ TFE/ACN | 55°C-60°C | TFSBr/ ACN | 1 | 60°C | 1 | 94 | 62 | 66 |
| 2 | K₂CO₃/ TFE/MCB | 40°C | TFSBr/ MCB | 1,5 | 40°C | 0,5 | 85 | 82 | 96 |
| 3 | K₂CO₃/ ODCB | 25°C | TFSBr/ TFE (1/2) | 1 | 25°C | 0,5 | 45 | 89* | 89 |
| 4 (comp.) | K₂CO₃/ TFE/H₂O | 40°C | TFSBr/ ODCB | 1,5 | 40°C | 0,7 | 44 | 27 | 61 |
| 5 | K₂CO₃/ xylènes | 25°C | TFSBr/ TFE (1/2) | 1,2 | 25°C | 0,8 | 56 | 95* | 95 |

| | | | | | | | | | |
|---|---|---|---|---|---|---|---|---|---|
| * = calculé par rapport au TFSBr. | | | | | | | | | |

### Exemple 6

Dans un réacteur en verre de 150 ml, on introduit sous azote :
- K₂CO₃ : 7 g (50,14 mmol)
- EtOH : 3,2 g (32 mmol)
- ODCB : 25 g

Le milieu hétérogène résultant est porté sous agitation à 25°C, puis le TFSBr (7 g, 32 mmol), dilué par EtOH (1,5 g, 31 mmol)), est ajouté pendant 1 h.

Après ajout, le milieu réactionnel est maintenu à 25°C pendant 1 h.

Les résultats obtenus sont les suivants :
- RT = 100 %,
- RR_{TfOEt} = 53 % (calculé par rapport au TFSBr).

### Exemples comparatifs 7 à 10

Dans un réacteur en verre de 150 ml, on introduit sous azote :
- TFE : 7,05 g (70,5 mmol)
- amine tertiaire NR₃ : 7,2 g (70,5 mmol)
- solvant : 14 g

La solution résultante est portée sous agitation à la température T mentionnée dans le tableau suivant (II), puis le TFSBr (15 g, 70,5 mmol), dilué ou non par un solvant (14 g), est ajouté pendant un temps t également mentionné dans le tableau (II)

Après ajout, le milieu réactionnel est maintenu à la température T' pendant un temps t' mentionné dans le tableau (II), puis l'on dose les réactifs et les produits obtenus par RMN ¹⁹F.

Le tableau suivant (II) rassemble les résultats correspondants :

**Tableau (II)**

| | REACTION | | | | MAINTIEN | | | | |
|---|---|---|---|---|---|---|---|---|---|
| Ex. | Réactifs en pied | T en pied (°C) | Réactifs coulés | Temps de coulée (h) | T' (°C) | t' (h) | TT (%) | RR (%) | RT (%) |
| 7 | TFE/NEt₃ | 0 | TFSBr | 2 | 0 | 1 | 100 | 12 | 12 |
| 8 | TFE/NEt₃ | 60 | TFSBr | 1,7 | 60 | 0,3 | 100 | 0 | 0 |
| 9 | TFE/NEt₃ /ACN | 60 | TFSBr/ACN | 1,5 | 60 | 0,3 | 100 | 0 | 0 |
| 10 | TFE /NEt₂i-Pr | 0 | TFSBr | 3 | 0 | 1 | 100 | 0 | 0 |

### Exemple 11

On reproduit l'exemple 6 à la seule différence que l'on met en oeuvre TFSCI (5,35 g, 32 mmol) pour conduire aux résultats suivants :
- TT = 100 %,
- RR_{TfOEt} = 51 % (calculé par rapport au TFSCl).

## Revendications

1. Procédé de sulfonylation d'un composé organique hydroxylé **caractérisé par le fait qu'**il comprend la réaction dudit composé, avec un agent de sulfonylation de formule (II), en milieu organique, en présence d'une base minérale hétérogène et d'un solvant organique apolaire : 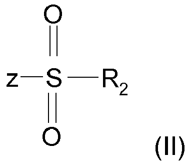 dans ladite formule (II) :
- R₂ représente un groupe hydrocarboné perfluoroalkyle CF₃,
- Z représente un atome de fluor, de chlore ou de brome.

2. Procédé selon la revendication 1 **caractérisé par le fait que** le composé organique hydroxylé répond à la formule (I) :
R¹-O-H (I)
dans ladite formule (I) :
- R₁ représente un groupe hydrocarboné, substitué ou non, comportant de 1 à 40 atomes de carbone qui peut être un groupe aliphatique acyclique saturé ou insaturé, linéaire ou ramifié ; un groupe carbocyclique ou hétérocyclique saturé, insaturé ou aromatique, monocyclique ou polycyclique ; un enchaînement des groupes précités.

3. Procédé selon la revendication 2 **caractérisé par le fait que** le composé organique hydroxylé est un alcool aliphatique qui répond à la formule (I) dans laquelle R₁ représente un groupe aliphatique acyclique saturé linéaire ou ramifié ayant de 1 à 12 atomes de carbone.

4. Procédé selon la revendication 2 **caractérisé par le fait que** le composé organique hydroxylé est un alcool aliphatique fluoré ou perfluoré répondant à la formule (la) :
R₁-O-H (la)
dans ladite formule (la), R₁ représente une chaîne alkyle fluorée ou perfluorée comprenant de 1 à 10 atomes de carbone et de 1 à 21 atomes de fluor.

5. Procédé selon la revendication 1 **caractérisé par le fait que** le composé organique hydroxylé est choisi parmi : l'éthanol, le 2,2,2-trifluoroéthanol, le 2,2-difluoroéthanol, le 1,1-difluoroéthanol, le pentafluoroéthanol, l'hexafluoroisopropanol, le pentafluorophénol, le p-nitrophénol, le p-trifluorométhylphénol.

6. Procédé selon la revendication 1 **caractérisé par le fait que** l'agent de trifluorométhanesulfonylation répond à la formule (II) dans laquelle Z est un atome de chlore ou de brome.

7. Procédé selon la revendication 1 **caractérisé par le fait que** l'agent de trifluorométhanesulfonylation est le fluorure de trifluorométhanesulfonyle, le chlorure de trifluorométhanesulfonyle ou le bromure de trifluorométhanesulfonyle.

8. Procédé selon l'une des revendications 1 à 7 **caractérisé par le fait que** le rapport entre le nombre de moles d'agent de trifluorométhanesulfonylation et le nombre de moles de composé organique hydroxylé varie entre 0,5 et 2.

9. Procédé selon l'une des revendications 1 à 8 **caractérisé par le fait que** la base est un hydroxyde d'ammonium ou d'un métal monovalent et/ou d'un métal bivalent; un carbonate ou un hydrogénocarbonate de métal alcalin ou alcalino-terreux.

10. Procédé selon la revendication 9 **caractérisé par le fait que** la base est le carbonate de sodium ou de potassium.

11. Procédé selon la revendication 1 **caractérisé par le fait que** la base est sous une forme solide, sous forme d'une poudre ou sous une forme broyée dans le cas des hydroxydes.

12. Procédé selon la revendication 1 **caractérisé par le fait que** la quantité de base mise en oeuvre est telle que le rapport entre le nombre de moles de base et le nombre de moles d'agent de trifluorométhanesulfonylation varie entre 1 et 2.

13. Procédé selon la revendication 1 **caractérisé par le fait que** la réaction est conduite en présence d'un solvant organique apolaire choisi parmi un hydrocarbure aliphatique, cycloaliphatique ou aromatique halogéné ou non.

14. Procédé selon l'une des revendications 1 à 13 **caractérisé par le fait que** l'on conduit la réaction dans des conditions anhydres.

15. Procédé selon l'une des revendications 1 à 14 **caractérisé par le fait que** la réaction est mise en oeuvre à une température comprise entre 0°C et la température de reflux du solvant organique.

16. Procédé selon l'une des revendications 1 à 15 **caractérisé par le fait qu'**il consiste à préparer un pied constitué par le composé organique hydroxylé et la base minérale puis d'introduire progressivement l'agent de trifluorométhanesulfonylation introduit dans le solvant organique.

17. Procédé selon l'une des revendications 1 à 16 **caractérisé par le fait qu'**il consiste à former un pied comprenant la base minérale et le solvant organique puis d'introduire progressivement l'agent de trifluorométhanesulfonylation et le composé organique hydroxylé.

18. Procédé selon l'une des revendications 1 à 17 **caractérisé par le fait que** l'on fait réagir le 2,2,2-trifluoroéthanol et le bromure de trifluorométhanesulfonyle.

## Patentansprüche

1. Verfahren zur Sulfonylierung einer organischen Hydroxylverbindung, **dadurch gekennzeichnet, dass** man die Verbindung in organischem Medium in Gegenwart einer heterogenen anorganischen Base und eines unpolaren organischen Lösungsmittels mit einem Sulfonylierungsmittel der Formel (II): 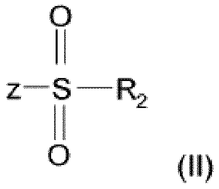 umsetzt, wobei in Formel (II):
- R₂ für eine CF₃-Perfluoralkylkohlenwasserstoffgruppe steht,
- Z für ein Fluor-, Chlor- oder Bromatom steht.

2. Verfahren nach Anspruch 1, **dadurch gekennzeichnet, dass** die organische Hydroxylverbindung der Formel (I) :
R₁-O-H (I)
entspricht, wobei in Formel (I):
- R₁ für eine gegebenenfalls substituierte Kohlenwasserstoffgruppe mit 1 bis 40 Kohlenstoffatomen, bei der es sich um eine lineare oder verzweigte, gesättigte oder ungesättigte acyclische aliphatische Gruppe, eine monocyclische oder polycyclische, gesättigte, ungesättigte oder aromatische carbocyclische oder heterocyclische Gruppe oder eine Verkettung der oben aufgeführten Gruppen handeln kann, steht.

3. Verfahren nach Anspruch 2, **dadurch gekennzeichnet, dass** es sich bei der organischen Hydroxyl-verbindung um einen aliphatischen Alkohol handelt, der der Formel (I) entspricht, wobei R₁ für eine lineare oder verzweigte gesättigte acyclische aliphatische Gruppe mit 1 bis 12 Kohlenstoffatomen steht.

4. Verfahren nach Anspruch 2, **dadurch gekennzeichnet, dass** es sich bei der organischen Hydroxylverbindung um einen fluorierten oder perfluorierten aliphatischen Alkohol handelt, der der Formel (Ia) :
R₁-O-H (Ia)
entspricht, wobei in Formel (Ia) R₁ für eine fluorierte oder perfluorierte Alkylkette mit 1 bis 10 Kohlenstoffatomen und 1 bis 21 Fluoratomen steht.

5. Verfahren nach Anspruch 1, **dadurch gekennzeichnet, dass** die organische Hydroxylverbindung aus Ethanol, 2,2,2-Trifluorethanol, 2,2-Difluor-ethanol, 1,1-Difluorethanol, Pentafluorethanol, Hexafluorisopropanol, Pentafluorphenol, p-Nitrophenol und p-Trifluormethylphenol ausgewählt wird.

6. Verfahren nach Anspruch 1, **dadurch gekennzeichnet, dass** das Trifluormethansulfonylierungsmittel der Formel (II) entspricht, wobei Z für ein Chlor- oder Bromatom steht.

7. Verfahren nach Anspruch 1, **dadurch gekennzeichnet, dass** es sich bei dem Trifluormethansulfonylierungsmittel um Trifluormethansulfonylfluorid, Trifluormethansulfonylchlorid oder Trifluormethansulfonylbromid handelt.

8. Verfahren nach einem der Ansprüche 1 bis 7, **dadurch gekennzeichnet, dass** das Verhältnis zwischen der Zahl der Mole des Trifluormethansulfonylierungsmittels und der Zahl der Mole der organischen Hydroxylverbindung zwischen 0,5 und 2 variiert.

9. Verfahren nach einem der Ansprüche 1 bis 8, **dadurch gekennzeichnet, dass** es sich bei der Base um ein Hydroxid von Ammonium oder einem einwertigen Metall und/oder einem zweiwertigen Metall oder ein Alkalimetall- oder Erdalkalimetallcarbonat oder -hydrogencarbonat handelt.

10. Verfahren nach Anspruch 9, **dadurch gekennzeichnet, dass** es sich bei der Base um Natrium- oder Kaliumcarbonat handelt.

11. Verfahren nach Anspruch 1, **dadurch gekennzeichnet, dass** die Base in fester Form, in Form eines Pulvers oder in gemahlener Form im Fall der Hydroxide vorliegt.

12. Verfahren nach Anspruch 1, **dadurch gekennzeichnet, dass** die Base in einer solchen Menge eingesetzt wird, dass das Verhältnis der Zahl der Mole der Base und der Zahl der Mole des Trifluormethansulfonylierungsmittels zwischen 1 und 2 variiert.

13. Verfahren nach Anspruch 1, **dadurch gekennzeichnet, dass** die Umsetzung in Gegenwart eines unpolaren organischen Lösungsmittels durchgeführt wird, das aus einem gegebenenfalls halogenierten aliphatischen, cycloaliphatischen oder aromatischen Kohlenwasserstoff ausgewählt wird.

14. Verfahren nach einem der Ansprüche 1 bis 13, **dadurch gekennzeichnet, dass** man die Umsetzung unter wasserfreien Bedingungen durchführt.

15. Verfahren nach einem der Ansprüche 1 bis 14, **dadurch gekennzeichnet, dass** man die Umsetzung bei einer Temperatur zwischen 0°C und der Rückflusstemperatur des organischen Lösungsmittels durchführt.

16. Verfahren nach einem der Ansprüche 1 bis 15, **dadurch gekennzeichnet, dass** man eine Vorlage herstellt, die aus der organischen Hydroxylverbindung und der anorganischen Base besteht, und dann allmählich das Trifluormethansulfonylierungsmittel in dem organischen Lösungsmittel einträgt.

17. Verfahren nach einem der Ansprüche 1 bis 16, **dadurch gekennzeichnet, dass** man eine Vorlage herstellt, die die anorganische Base und das organische Lösungsmittel umfasst, und dann allmählich das Trifluormethansulfonylierungsmittel und die organische Hydroxylverbindung einträgt.

18. Verfahren nach einem der Ansprüche 1 bis 17, **dadurch gekennzeichnet, dass** man 2,2,2-Trifluorethanol und Trifluormethansulfonylbromid umsetzt.

## Claims

1. Process for the sulfonylation of a hydroxylated organic compound, **characterized in that** it comprises the reaction of said compound with a sulfonylating agent of formula II, in an organic medium, in the presence of a heterogeneous inorganic base and of a nonpolar organic solvent: 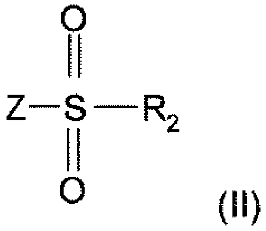 in said formula (II) :
- R₂ representing a perfluoroalkyl hydrocarbon group CF₃,
- Z representing a fluorine, chlorine or bromine atom.

2. Process according to Claim 1, **characterized in that** the hydroxylated organic compound corresponds to the formula (I):
R₁- 0 - H (I)
in said formula (I) :
- R₁ representing a substituted or unsubstituted hydrocarbon group comprising from 1 to 40 carbon atoms which can be a saturated or unsaturated and linear or branched acyclic aliphatic group, a saturated, unsaturated or aromatic and monocyclic or polycyclic carbocyclic or heterocyclic group, or a sequence of the abovementioned groups.

3. Process according to Claim 2, **characterized in that** the hydroxylated organic compound is an aliphatic alcohol which corresponds to the formula (I) in which R₁ represents a saturated and linear or branched acyclic aliphatic group having from 1 to 12 carbon atoms.

4. Process according to Claim 2, **characterized in that** the hydroxylated organic compound is a fluorinated or perfluorinated aliphatic alcohol corresponding to the formula (Ia):
R₁ - 0 - H (Ia)
in said formula (Ia) R₁ representing a fluorinated or perfluorinated alkyl chain comprising from 1 to 10 carbon atoms and from 1 to 21 fluorine atoms.

5. Process according to Claim 1, **characterized in that** the hydroxylated organic compound is chosen from ethanol, 2,2,2-trifluoroethanol, 2,2-difluoroethanol, 1,1-difluoroethanol, pentafluoroethanol, hexafluoroisopropanol, pentafluorophenol, p-nitrophenol or p-(trifluoromethyl)phenol.

6. Process according to Claim 1, **characterized in that** the trifluoromethanesulfonylating agent corresponds to the formula (II) in which Z is a chlorine or bromine atom.

7. Process according to Claim 1, **characterized in that** the trifluoromethanesulfonylating agent is trifluoromethanesulfonyl fluoride, trifluoromethanesulfonyl chloride or trifluoromethanesulfonyl bromide.

8. Process according to one of Claims 1 to 7, **characterized in that** the ratio of the number of moles of trifluoromethanesulfonylating agent to the number of moles of hydroxylated organic compound varies between 0.5 and 2.

9. Process according to one of Claims 1 to 8, **characterized in that** the base is a hydroxide of ammonium or of a monovalent metal and/or of a divalent metal, or an alkali metal or alkaline earth metal carbonate or hydrogencarbonate.

10. Process according to Claim 9, **characterized in that** the base is sodium carbonate or potassium carbonate.

11. Process according to Claim 1, **characterized in that** the base is in a solid form, in the form of a powder or in a ground form in the case of the hydroxides.

12. Process according to Claim 1, **characterized in that** the amount of base employed is such that the ratio of the number of moles of base to the number of moles of trifluoromethanesulfonylating agent varies between 1 and 2.

13. Process according to Claim 1, **characterized in that** the reaction is carried out in the presence of a nonpolar organic solvent chosen from a halogenated or nonhalogenated aliphatic, cycloaliphatic or aromatic hydrocarbon.

14. Process according to one of Claims 1 to 13, **characterized in that** the reaction is carried out under anhydrous conditions.

15. Process according to one of Claims 1 to 14, **characterized in that** the reaction is carried out at a temperature of between 0°C and the reflux temperature of the organic solvent.

16. Process according to one of Claims 1 to 15, **characterized in that** it consists in preparing a heel composed of the hydroxylated organic compound and the inorganic base and in then gradually introducing the trifluoromethanesulfonylating agent introduced in the organic solvent.

17. Process according to one of Claims 1 to 16, **characterized in that** it consists in forming a heel comprising the inorganic base and the organic solvent and in then gradually introducing the trifluoromethanesulfonylating agent and the hydroxylated organic compound.

18. Process according to one of Claims 1 to 17, **characterized in that** 2,2,2-trifluoroethanol and trifluoromethanesulfonyl bromide are reacted.
